# EUROPEAN PATENT SPECIFICATION

(11) **EP 2 168 551 B1**
(45) Date of publication and mention of the grant of the patent: **13.07.2016**
(21) Application number: 09011929.8
(22) Date of filing: 18.09.2009
(51) Int. Cl.: A61H 23/02, A61H 23/04, A61H 1/00, A61H 9/00, A47C 3/02, A61M 21/02, A47C 3/025, A61M 21/00

(54) **Relaxation apparatus**
Entspannungseinrichtung
Appareil de relaxation

(30) Priority: 30.09.2008 JP 2008254901
(43) Date of publication of application: 31.03.2010
(73) Proprietor: Panasonic Intellectual Property Management Co., Ltd., Osaka 540-6207 (JP)
(72) Inventor: Takahashi, Tatsuya, Kadoma-shi, Osaka (JP); Michimori, Akihiro, Kadoma-shi, Osaka (JP); Morikawa, Daisuke, Kadoma-shi, Osaka (JP); Nishimura, Yoshinari, Kadoma-shi, Osaka (JP)
(74) Representative: Samson & Partner Patentanwälte mbB

(56) References cited:
- DE-U1- 20 001 337
- JP-A- 2000 300 374
- US-A- 4 656 680
- US-A- 5 520 614
- US-B1- 6 494 850

## Description

### Field of the Invention

The present invention relates to an apparatus for providing a user with a relaxing effect.

### Background of the Invention

A conventional relaxation apparatus, which provides a user seating on a chair-shaped body-supporting member with relaxing effect by rocking the user's body, is described in Japanese Patent Laid-open Publication No. 2000-42112 (hereinafter, referred to as the cited reference).

The relaxation apparatus in the cited reference has a rocking unit (a rocking mechanism in the cited reference) which rocks a body-supporting member back and forth and hence provides a user with a relaxing effect by rocking the body-supporting member with the rocking unit at a frequency of 1 Hz or less while gradually changing the frequency with fluctuations of 0.2 Hz or less.

In the above-mentioned relaxation apparatus, because the user sitting on the body-supporting member is subjected to a rocking motion of his/her body, particularly, when the frequency of the rocking motion is high, he/she is prone to discomfort arising from a strong stimulation on the semicircular canal (in the vestibular organ) responsible for the sense of equilibrium, possibly inducing diseases such as a nausea. As such, the relaxation effect brought by the rocking motion gets reduced, and hence its resolution is in great demand.

DE 200 01 337 U1, US 6 494 850 B1 , US 5,520,614 A, US 4,656,680 A and JP 2000 300374 all disclose a relaxation apparatus according to the preamble of claim 1.

### Summary of the Invention

In view of the above, the present invention provides a relaxation apparatus capable of giving a user a relaxation effect in a more effective way.

While the invention is defined in the independent claim 1, further aspects of the invention are set forth in the dependent claims, the drawings and the following description.

In accordance with an aspect of the present invention, there is provided a relaxation apparatus including a body-supporting unit for supporting the body of a user, a rocking unit for rocking the body-supporting unit and a controller for controlling the rocking unit to rock the body-supporting unit, characterized in that: the controller controls the rocking unit to rock the user so that the vestibular organ or a body part equivalent thereto of the user is moved within a rocking range with a radius of 50 mm therearound.

The controller may control the rocking unit to rock the body-supporting unit back and forth.

The relaxation apparatus may further include a position-recognition unit for recognizing a position of the vestibular organ or the body part equivalent thereto of the user. In this case, the controller controls the rocking unit so that the position of the vestibular organ or the body part equivalent thereto recognized by the position-recognition unit falls within the rocking range.

The position-recognition unit may define the position of the vestibular organ or the body part equivalent thereto of the user based on physical attribute information of the user.

Here, the physical attribute information stands for a height or a sitting height of the user. In this case, the position of the vestibular organ or the body part equivalent thereto of the user can be defined easily with a simple configuration, without having to measure the position of the vestibular organ or the body part equivalent thereto of the user.

In accordance with the present invention, the relaxation apparatus is capable of giving an effective relaxation effect to a user.

### Brief Description of the Drawings

Fig. 1 shows a schematic configuration of a relaxation apparatus in accordance with an embodiment of the present invention.
Fig. 2 depicts a graph for explaining frequency and amplitude of a rocking motion of a body-supporting member of the relaxation apparatus not covered by the claims.
Fig. 3 depicts a graph for explaining the relationship between the degree of comfort to a user and the range of movement of the vestibular organ.
Fig. 4 is a schematic view showing the position of the tragus of a user.
Fig. 5 is a schematic view showing the tragus and vestibular organ of a user.
Fig. 6 depicts a graph for explaining the relationship between the degree of comfort to a user and the range of movement of the vestibular organ in another example.

### Detailed Description of the Embodiments

Hereinafter, embodiments of the present invention will be described in detail with reference to the accompanying drawings.

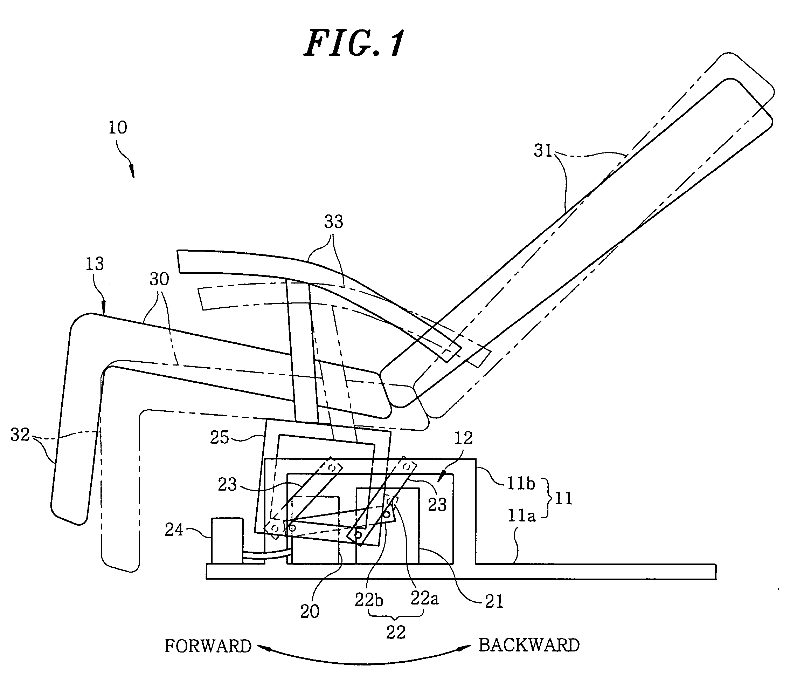

Fig. 1 shows a schematic configuration of a relaxation apparatus in accordance with an embodiment of the present invention. The relaxation apparatus 10 includes a base 11 having a bottom portion 11a placed on a floor (not shown), a rocking mechanism 12 as a rocking unit provided to the base 11 and a body-supporting member 13 as a body-supporting unit driven by the rocking mechanism 12.

The rocking mechanism 12 has a motor 20, a reduction gear 21, a crank mechanism 22 and a plurality of, e.g., two, link members 23.

The motor 20 is provided on the bottom portion 11a of the base 11. The operation of the motor 20 is controlled by a controller 24 provided on the bottom portion 11a of the base 11. The reduction gear 21 is provided on the bottom portion 11a of the base 11. The reduction gear 21 is operationally coupled with the motor 20 and serves to reduce the power of the motor 20.

The crank mechanism 22 has two connecting rods 22a and 22b, and converts the rotating motion of the reduction gear 21 into a large circular motion. The base end of the connecting rod 22a is connected to and rotates with an output shaft of the reduction gear 21, while its leading end is ratatably connected to the base end of the connecting rod 22b. The leading end of the connecting rod 22b is connected to the lower portion of a rectangular frame 25 to which the body-supporting member 13 is fixed.

The base 11 also has a support frame 11b protruded upward from the bottom portion 11a. The base ends of the linkages 23 are rotatably connected to the upper portion of the support frame 11b at an interval, while their leading ends 22b are rotatably connected to the lower portion of the rectangular frame 25. The linkages 23 rotate around their base ends and, therefore, the body-supporting member 13, fixed to the rectangular frame 25, can be rocked back and forth by the power delivered from the crank mechanism 22, like a rocking chair. In the present embodiment of the invention, the movement path of the body-supporting member 13 from the rear to the front is referred to as a forward path and that from the front to the rear is referred to as a backward path.

The body-supporting member 13 of a chair shape includes a seat portion 30 which is fixed to an upper portion of the rectangular frame 25 so that they can move together, a back portion attached to the rear side of the seat portion 30 so that it can recline about the rear of the seat portion 30, an ottoman fixed at the front side of the seat portion 30 and two arm rests 33 fixed at both sides of the seat portion 30.

The back portion 31 can be reclined suitably by a reclining mechanism (not shown), which is controlled by the controller 24.

Hereinafter, the operation of the relaxation apparatus 10 configured as described above will be described with reference to Figs. 1 to 4.

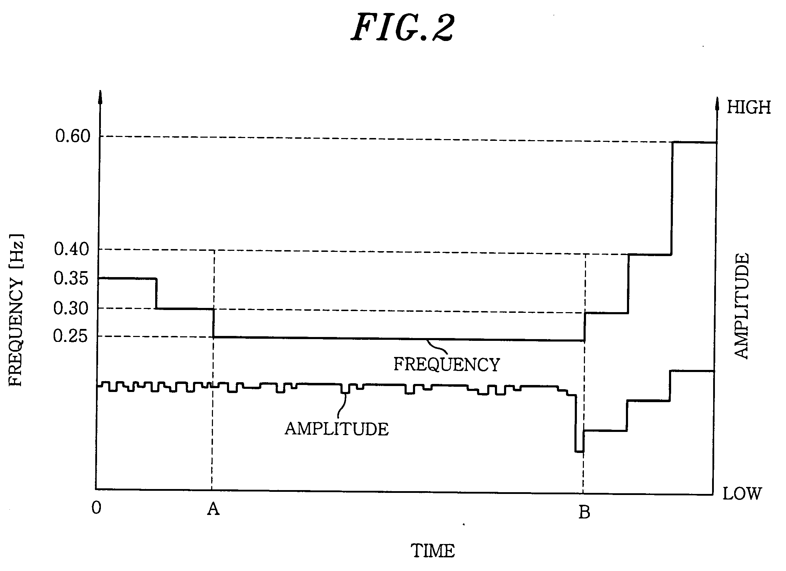

In the controller 24, several modes are provided including a single mode in which a relaxation rocking of the body-supporting member 13 is carried out by driving the rocking mechanism 12 and a combination mode in which the relaxation rocking is carried out in combination with operation of the respective air bags 40 to 48 and reclining of the back portion 31. Among them, in a relaxation-rocking mode having a refresh effect, a "relaxation rocking" is initiated to give a relaxation effect to a user for a predetermined period of time, after which the operation mode is switched from the "relaxation rocking" to "refresh rocking" until the operation mode ends. Fig. 2 shows the variations of amplitude and frequency with time of the motion of the body-supporting member 13 driven by the rocking mechanism 12 in the relaxation-rocking mode.

### (Relaxation rocking)

At first, the controller 24 controls the reclining mechanism to recline the back portion 31 by a specified degree (for example, Δθ = 30°) from a normal state (for example, at θ = 120°) to a reclined state (for example, at θ = 150°), where θ denotes the angle the back portion 31 makes with the seat portion 30. During the reclining, the speed at which the back 31 is reclined is controlled to have a gradual decrease. In order to lead a user to be from an awakened state to a relaxed state, the controller 24 controls the motor 20 to decrease the frequency of rocking of the body-supporting member 13 gradually, for example, from its initial value 0.35 Hz to the final 0.2 Hz in a piecewise manner by a decrement of 0.05 Hz until a predetermined time denoted by A in Fig. 2. At the same time, the motor is controlled to introduce fluctuations to the amplitude of the rocking motion.

Until a predetermined time B after the time A has lapsed, the controller 24 controls the motor 20 to generate fluctuations in the amplitude of the body-supporting member 13 at the minimum value of the frequency, 0.2 Hz in the present embodiment, in order to let the user rest in sleep in the relaxation state.

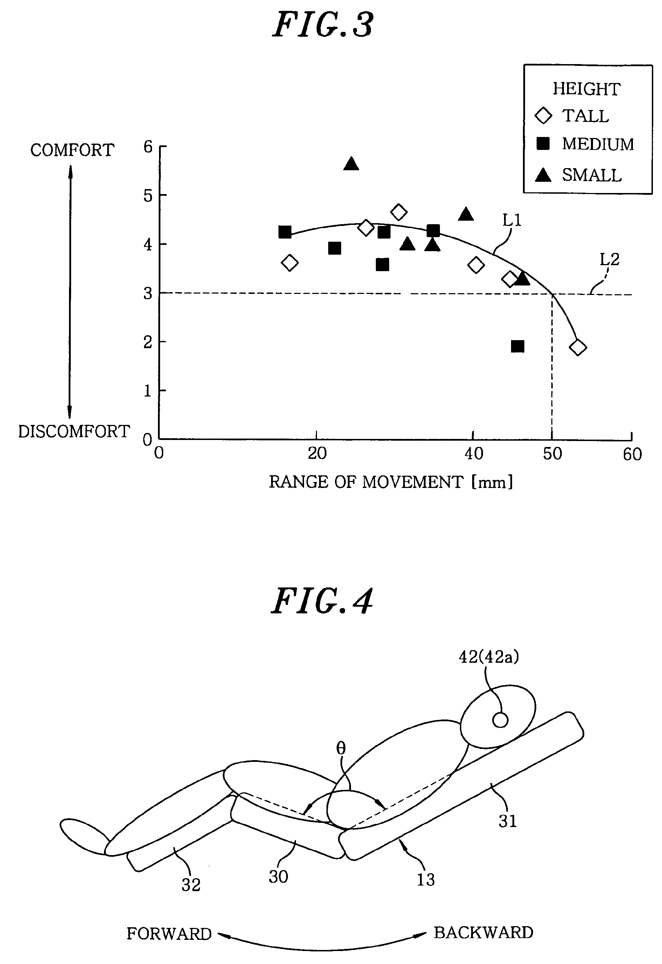

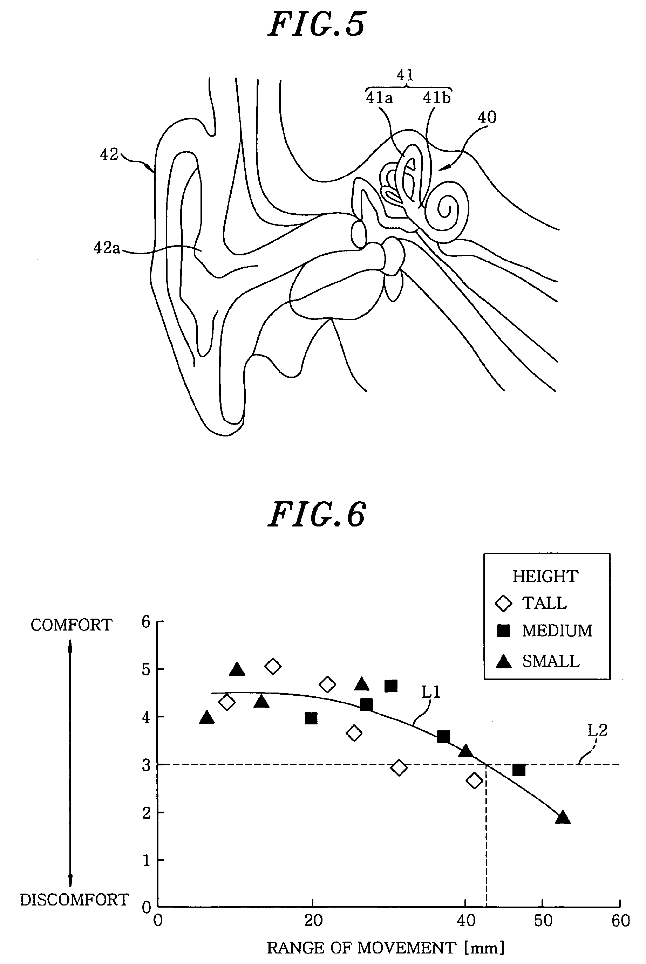

Here, with regard to the aforementioned relaxation rocking motion, an experiment has been carried out in order to investigate the region of movement that can give a more effective relaxation effect by suppressing a so-called nausea. First, the experiment investigates, based on the fact that the vestibular organ 41 of a user is responsible for the sense of equilibrium in the internal ear 40 of the user (see Fig. 5), the relationship of the degree of comfort to the user with the range of movement of his/her vestibular organ 41, evaluating the degree of comfort in seven levels, 0 to 6, from "discomfort" to "comfort" as shown in Fig. 3. Besides, the vestibular organ 41 includes the semicircular canal 41a and the otolith 41b (see Fig. 5).

In addition, the evaluation experiment has been carried out at a frequency 0.25 Hz of the rocking motion and for an angle of reclining θ = 150°. In the experiment, the difference in the position of the vestibular organ 41 due to the difference in physical features (the height, in particular) has been taken into consideration by classifying the height into three categories, tall, medium and little, corresponding respectively to the cases greater than or equal to 180 cm, less than 180 cm and greater than or equal to 170 cm, and less than 170 cm. Besides, the range of movement of the vestibular organ 41 is regarded as being equivalent to that of the tragus 42a of the external ear 42 of the user (see Figs. 4 and 5).

The set of all data to estimate the relationship of the degree of comfort to a user with the range of movement of the vestibular organ 41 (the tragus 42a) during a rocking motion is approximated by a curve L₁ in Fig. 3. The curve L₁ intersects the horizontal line L₂, representing the midpoint 3 between the comfort and discomfort, at a point corresponding to a range of movement 50 mm of the vestibular organ 41 (the tragus 42a). Hence, in the present embodiment, the controller 24 controls the rocking unit 12 so that the vestibular organ (the tragus 42a) moved by the rocking motion remains within a range with a radius of 50 mm around the vestibular organ (the tragus 42a) when the angle of reclining is θ = 150° and the frequency of the rocking motion is 0.25 Hz (between the predetermined times A and B). It follows that the discomfort to the user is suppressed, providing with a more effective relaxation effect.

### (Refresh rocking)

In this example not covered by the claims, after the lapse of time B, the controller 24 controls the motor 24 to increase the frequency of the rocking motion in a piecewise manner, for example, from 0.2 Hz to 0.3 Hz, 0.4 Hz and 0.6 Hz, in order to induce a user in the sleep (or relaxation) state to an awakened state. At this time, the controller 24 controls the frequency change timings, for example, such that the frequency changes every ten round trips the rocking motion of the body-supporting member 13 makes. Further, the controller 24 controls the motor 20 such that the amplitude increases stepwise in synchronization with the frequency change timings.

Next, the characteristic effects of the present embodiment will be described.
(1) The controller 24 controls the rocking unit 12 so that the tragus 42a, a body part equivalent to the vestibular organ 41, of a user is moved within a rocking range with a radius of 50 mm therearound. From the relationship between the degree of comfort with the movement of the vestibular organ 41 (or the tragus 42a, a body part equivalent thereto) in Fig. 3, it can be seen that the discomfort to the user is suppressed and so is the possibility of having a nausea, providing him/her with a more effective relaxation effect.
(2) The controller 24 controls the rocking unit 12 so that the body-supporting member 13 rocks back and forth. That is, the discomfort to a user gets suppressed by rocking of the body-supporting member 13 only back and forth, providing him/her with a more effective relaxation effect.

The embodiment of the present invention may be modified as described below.

In the embodiment described above, the controller 24 controls the rocking unit 12 so that the tragus 42a, a body part equivalent to the vestibular organ 41, of a user is moved within a rocking range with a radius of 50 mm therearound at an angle of reclining θ = 150°. In a modified configuration, the relaxation rocking can be carried out at a different angle of reclining, for example, θ = 135°. When the angle of reclining is θ = 135° in particular, it is more desirable from the viewpoint of suppressing the discomfort to carry out the rocking motion so that the tragus 42a (the vestibular organ 41) is moved within a rocking range with a radius of less than 50 mm (for example, 40 mm).

In the example described above, the rocking motion is carried out so that the tragus 42a (the vestibular organ 41) remains within a specific rocking range (a radius of 50 mm) therearound at a frequency 0.25 Hz. However, it is also possible to carry out so that the tragus 42a (the vestibular organ 41) remains within a specific rocking range therearound at frequencies different than 0.25 Hz. In this case, according to the invention, the range of motion of the tragus 42a (the vestibular organ 41) is reduced as the frequency gets higher.

Although, in the embodiment described in the above, it is configured that the body-supporting member 13 is rocked back and forth, the present invention is not limited thereto. For example, a side-to-side rocking or a combination of a side-to-side rocking with a back-and-forth rocking may be employed. In addition, although it is desirable to carry out a rocking motion within a radius of 50 mm around the tragus 42a (the vestibular organ 41) of a user, a different range may be used if the movement range of the tragus 42a (the vestibular organ 41) is small.

Although it is not mentioned explicitly in the embodiment described above, for example, the relaxation apparatus may include a position-recognition unit for recognizing the position of the vestibular organ 41 (the tragus 42a) of a user and the controller 24 may control the rocking unit 12 so that the position of the vestibular organ 41 (the tragus 42a) recognized by this position-recognition unit falls within its movement range (50 mm). In other words, with the position-recognizing part recognizing the position of the vestibular organ 41 (the tragus 42a) of a user, it becomes possible for the controller 24 to perform a control corresponding to the difference in the physical attributes (the height in particular), providing the user with a more effective relaxation effect.

In addition to the above configuration, the relaxation apparatus may include an input unit for inputting physical attributes such as the height or seating height of a user and the position of the vestibular organ 41 (the tragus 42a) may be defined by the position-recognition unit based on the inputted information. In this case, the position of the vestibular organ 41 (the tragus 42a) can be defined easily in a simple configuration because, if the user inputs his/her physical attributes, the position of the vestibular organ 41 (the tragus 42a) can be specified by the statistical data previously obtained from experiments without having to carry out measurements of the position of the vestibular organ 41 (the tragus 42a) by the position-recognition unit.

Although, in the embodiment described above, the tragus 42a is selected for a body part corresponding to the vestibular organ 41, the present invention is not limited thereto, but the external ear 42 may be employed for example.

In the embodiment described above, the controller 24 makes changes in control over the respective parts at the predetermined times A and B. In a modified configuration, for example, there may be provided a switch capable of changing the control of the respective parts by the controller 24 so that a user can selectively control the respective parts by using the switch as needed.

In the embodiment described above, during the relaxation rocking, the controller 24 controls the motor 20 to change the frequency of the rocking motion of the body-supporting member 13 (the rectangular frame 25) in a range from 0.35 Hz to 0.2 Hz. However, controller 24 may control the motor 20 to change the frequency of the rocking motion of the body-supporting member 13 in other frequency bands for the relaxation rocking motion.

In the embodiment described above, during the refresh rocking, the controller 24 controls the motor 20 to change the frequency of the rocking motion of the body-supporting member 13 (the rectangular frame 25) from 0.25 Hz to 0.6 Hz stepwise. However, other frequency bands for the refresh rocking motion may be used.

Although it is not mentioned explicitly in the embodiment described above, the respective parts of the body-supporting member 13 may include air bags to yield a relaxation effect such as massaging by appropriately expanding and contracting or a refresh effect by stimulations arising from expansion motion.

In the embodiment described above, a relaxation rocking motion is followed by a refresh rocking motion. In a modified configuration, however, only a relaxation rocking motion may be carried out.

## Claims

1. A relaxation apparatus (10) comprising a body-supporting unit (13) for supporting a body of a user, a rocking unit (12) for rocking the body-supporting unit (13) and a controller (24) for controlling the rocking unit (12) to rock the body-supporting unit (13), **characterized in that**:
the controller (24) is adapted to control the rocking unit (12) to rock the user so that in use the vestibular organ (41) or a body part (42a) equivalent in position thereto of the user is moved within a rocking range of a specific radius,
wherein the rocking range of the vestibular organ (41) or the body part (42a) equivalent in position thereto is reduced as a rocking frequency of the body-supporting unit (13) gets higher.

2. The relaxation apparatus (10) of claim 1, wherein the controller (24) is adapted to control the rocking unit (12) to rock the body-supporting unit (13) back and forth.

3. The relaxation apparatus (10) of claim 1 or 2, further comprising a position-recognition unit for recognizing a current position of the vestibular organ (41) or the body part (42a) equivalent in position thereto of the user; and
wherein the controller (24) is adapted to control the rocking unit (12) so that the current position of the vestibular organ (41) or the body part (42a) equivalent in position thereto recognized by the position-recognition unit falls within the rocking range.

4. The relaxation apparatus (10) of claim 3, wherein the position-recognition unit defines the current position of the vestibular organ (41) or the body part (42a) equivalent in position thereto of the user based on physical attribute information of the user.

5. The relaxation apparatus (10) of claim 1, wherein the specific radius is 50 mm when the rocking frequency is 0.25 Hz.

## Patentansprüche

1. Entspannungsvorrichtung (10) umfassend eine Körperlagerungseinheit (13) zum Lagern eines Körpers eines Nutzers, eine Schaukeleinheit (12) zum Schaukeln der Körperlagerungseinheit (13) und eine Steuervorrichtung (24) zum Steuern der Schaukeleinheit (12), um die Körperlagerungseinheit (13) zu schaukeln, **dadurch gekennzeichnet, dass**:
die Steuervorrichtung (24) ausgelegt ist, um die Schaukeleinheit (12) derart zu steuern, um den Nutzer derart zu schaukeln, so dass in Betrieb das Vestibularorgan (41) oder ein Körperteil (42a), das sich in einer äquivalenten Position dazu befindet, des Nutzers innerhalb eines Schaukelbereichs eines bestimmten Radius bewegt wird,
wobei der Schaukelbereich des Vestibularorgans (41) oder des in Position dazu äquivalenten Körperteils (42a) reduziert wird, wenn eine Schaukelfrequenz der Körperlagerungseinheit (13) sich erhöht.

2. Entspannungsvorrichtung (10) gemäß Anspruch 1, bei der die Steuervorrichtung (24) ausgelegt ist, um die Schaukeleinheit (12) zu steuern, um die Körperlagerungseinheit (13) nach vorne und nach hinten zu schaukeln.

3. Entspannungsvorrichtung (10) gemäß Anspruch 1 oder 2, weiter umfassend eine Positionserkennungseinheit zum Erkennen einer gegenwärtigen Position des Vestibularorgans (41) oder des in Position dazu äquivalenten Körperteils (42a) des Nutzers; und
wobei die Steuervorrichtung (24) ausgelegt ist, um die Schaukeleinheit (12) derart zu steuern, dass die gegenwärtige Position des Vestibularorgans (41) oder des in Position dazu äquivalenten Körperteils (42a), die über die Positionserkennungseinheit erkannt wird, in den Schaukelbereich fällt.

4. Entspannungsvorrichtung (10) gemäß Anspruch 3, bei der die Positionserkennungseinheit die gegenwärtige Position des Vestibularorgans (41) oder des in Position dazu äquivalenten Körperteils (42a) des Nutzers basierend auf einer physikalischen Attributinformation des Nutzers basierend definiert.

5. Entspannungsvorrichtung (10) gemäß Anspruch 1, bei der der spezifische Radius 50 mm beträgt, wenn die Schaukelfrequenz 0,25 Hertz beträgt.

## Revendications

1. Appareil de relaxation (10) comprenant une unité de soutien (13) de corps pour soutenir un corps d'un utilisateur, une unité de balancement (12) pour balancer l'unité de soutien (13) de corps et un dispositif de commande (24) pour commander l'unité de balancement (12) afin de balancer l'unité de soutien (13) de corps, **caractérisé en ce que** :
le dispositif de commande (24) est adapté pour commander l'unité de balancement (12) pour balancer l'utilisateur de telle sorte que, lors de l'utilisation, l'organe vestibulaire (41), ou une partie corporelle (42a) équivalant en position à celui-ci, de l'utilisateur est déplacé dans une portée de balancement d'un rayon spécifique,
dans lequel la portée de balancement de l'organe vestibulaire (41), ou de la partie corporelle (42a) équivalant en position à celui-ci, est réduite à mesure qu'une fréquence de balancement de l'unité de soutien (13) de corps devient plus élevée.

2. Appareil de relaxation (10) selon la revendication 1, dans lequel le dispositif de commande (24) est adapté pour commander l'unité de balancement (12) pour balancer l'unité de soutien (13) de corps d'avant en arrière.

3. Appareil de relaxation (10) selon la revendication 1 ou 2, comprenant en outre une unité de reconnaissance de position pour reconnaître une position actuelle de l'organe vestibulaire (41), ou de la partie corporelle (42a) équivalant en position à celui-ci, de l'utilisateur ; et
dans lequel le dispositif de commande (24) est adapté pour commander l'unité de balancement (12) de telle sorte que la position actuelle de l'organe vestibulaire (41), ou de la partie corporelle (42a) équivalant en position à celui-ci, reconnue par l'unité de reconnaissance de position tombe dans la portée de balancement.

4. Appareil de relaxation (10) selon la revendication 3, dans lequel l'unité de reconnaissance de position définit la position actuelle de l'organe vestibulaire (41), ou de la partie corporelle (42a) équivalant en position à celui-ci, de l'utilisateur sur la base d'informations d'attributs physiques de l'utilisateur.

5. Appareil de relaxation (10) selon la revendication 1, le rayon spécifique est de 50 mm quand la fréquence de balancement est de 0,25 Hz.
